(19) Europäisches Patentamt
European Patent Office
Office européen des brevets

(11) EP 3 799 142 A1

(12) EUROPEAN PATENT APPLICATION
published in accordance with Art. 153(4) EPC

(43) Date of publication:
**31.03.2021 Bulletin 2021/13**

(21) Application number: **19852497.7**

(22) Date of filing: **16.08.2019**

(51) Int Cl.:
***H01L 51/50*** *(2006.01)* ***H01L 51/56*** *(2006.01)*
***H01L 51/00*** *(2006.01)*

(86) International application number:
**PCT/KR2019/010418**

(87) International publication number:
**WO 2020/040487 (27.02.2020 Gazette 2020/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.08.2018 KR 20180099501**

(71) Applicant: **LG CHEM, LTD.**
**Yeongdeungpo-gu,**
**Seoul 07336 (KR)**

(72) Inventors:
- **CHUN, Minseung**
**Daejeon 34122 (KR)**
- **KIM, Seong So**
**Daejeon 34122 (KR)**
- **HA, Jae Seung**
**Daejeon 34122 (KR)**

(74) Representative: **Hoffmann Eitle**
**Patent- und Rechtsanwälte PartmbB**
**Arabellastraße 30**
**81925 München (DE)**

# (54) ORGANIC LIGHT EMITTING DIODE

(57) The present disclosure provides an organic light emitting device having low driving voltage, high luminous efficiency and long lifetime characteristics.

[FIG. 1]

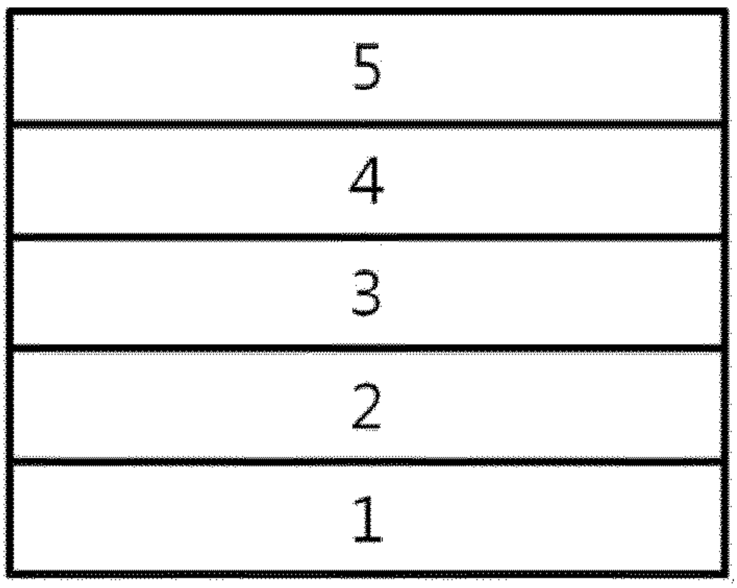




Printed by Jouve, 75001 PARIS (FR)

## Description

### [TECHNICAL FIELD]

CROSS-REFERENCE TO RELATED APPLICATION(S)

This application claims the benefit of Korean Patent Application No. 10-2018-0099501 filed on August 24, 2018 with the Korean Intellectual Property Office, the disclosure of which is incorporated herein by reference in its entirety.

The present disclosure relates to an organic light emitting device having low driving voltage, high luminous efficiency and long lifetime characteristics.

### [Background Art]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for the organic materials used in the organic light emitting devices as described above.

Meanwhile, most of the organic light emitting devices that are commercialized to date are manufactured by a deposition process. However, since the deposition process has a problem that the production cost is high, an organic light emitting device using a solution process has recently been developed to replace the deposition process. In the initial stage of development, attempts have been made to develop organic light emitting devices by coating all organic light emitting device layers by a solution process, but current technology has limitations. Therefore, only HIL, HTL, and EML are processed in a layer device structure by a solution process, and a hybrid process utilizing traditional deposition processes is being studied as a subsequent process.

The organic light-emitting device manufactured by such a solution process has different hole transport characteristics and/or electron transport characteristics as compared with an organic light emitting device manufactured by a conventional deposition process. Thus, when the material for forming the electron transport layer (ETL) is selected by a conventional method, there is a disadvantage in that the lifetime of the organic light emitting device is shortened.

In this regard, the present inventors have confirmed that the above problems are solved when a material for forming an electron transport layer that satisfies certain conditions is used at the time of manufacturing an organic light emitting device by a solution process, and completed the present disclosure.

### [Prior Art Literature]

### [Patent Literature]

(Patent Literature 1) Korean Unexamined Patent Publication No. 10-2000-0051826

### [DETAILED DESCRIPTION OF THE INVENTION]

### [Technical Problem]

It is an object of the present disclosure to provide an organic light emitting device having long lifetime characteristics.

### [Technical Solution]

In order to achieve the above object, the present disclosure provides an organic light emitting device:

An organic light emitting device comprising: an anode; a cathode that is provided opposite to the anode; a light emitting layer that is provided between the anode and the cathode; and an electron transport layer that is provided between the light emitting layer and the cathode,
wherein an electron mobility of a material constituting the electron transport layer is $10^{-5}$ $cm^2/Vs$ or less.

**[ADVANTAGEOUS EFFECTS]**

The organic light emitting device can have low driving voltage, high luminous efficiency, and particularly, long lifetime characteristic by using a material forming an electron transport layer that satisfies certain conditions when manufacturing an organic light-emitting device by a solution process.

**[BRIEF DESCRIPTION OF THE DRAWINGS]**

FIG. 1 shows an example of an organic light emitting device comprising a substrate 1, an anode 2, a light emitting layer 3, an electron transport layer 4 and a cathode 5.
FIG. 2 shows an example of an organic light emitting device comprising a substrate 1, an anode 2, a hole injection layer 6, a hole transport layer 7, a light emitting layer 3, an electron transport layer 4, an electron injection layer 8, and a cathode 5.

**[DETAILED DESCRIPTION OF THE EMBODIMENTS]**

Hereinafter, embodiments of the present disclosure will be described in more detail to facilitate understanding of the invention.

**(Definition of Terms)**

As used herein, the notation

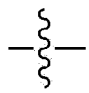

means a bond linked to another substituent group.
As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent to which two or more substituents of the above-exemplified substituents are connected. For example, "a substituent in which two or more substituents are connected" may be a biphenyl group. Namely, a biphenyl group may be an aryl group, or it may be interpreted as a substituent in which two phenyl groups are connected.
In the present disclosure, the carbon number of a carbonyl group is not particularly limited, but is preferably 1 to 40. Specifically, the carbonyl group may be a compound having the following structural formulas, but is not limited thereto.

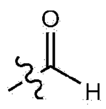
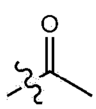
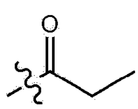
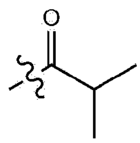

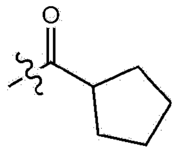 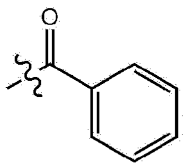 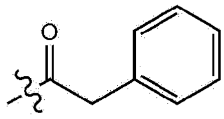

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a compound having the following structural formulas, but is not limited thereto.

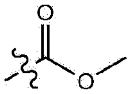 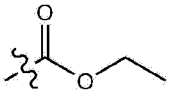 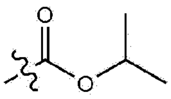 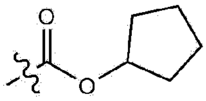 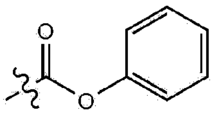

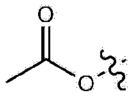 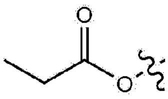 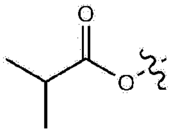 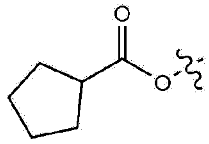 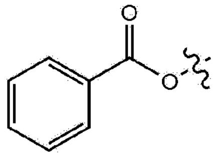

In the present disclosure, the carbon number of an imide group is not particularly limited, but is preferably 1 to 25. Specifically, the imide group may be a compound having the following structural formulas, but is not limited thereto.

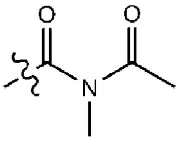 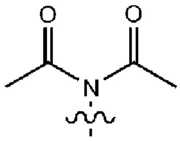 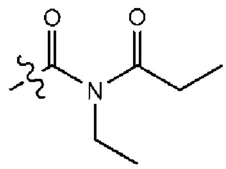

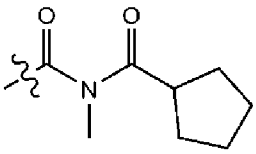 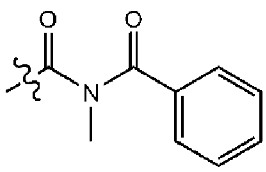

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the

alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthryl group, a pyrenyl group, a perylenyl group, a chrysenyl group, or the like, but is not limited thereto.

In the present disclosure, the fluorenyl group may be substituted, and two substituents may be linked with each other to form a spiro structure. In the case where the fluorenyl group is substituted,

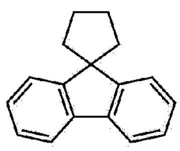 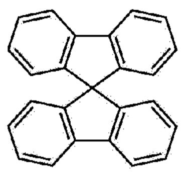 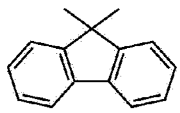 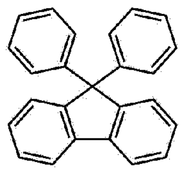 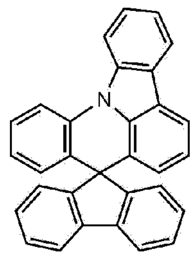

and the like can be formed. However, the structure is not limited thereto.

In the present disclosure, a heterocyclic group is a heterocyclic group containing one or more of O, N, Si and S as a heteroatom, and the carbon number thereof is not particularly limited, but is preferably 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group and the arylamine group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can be applied to the aforementioned description of the heterocyclic group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heteroaryl group can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heterocyclic group can be applied, except that the heteroaryl group is not a monovalent group but formed by combining two substituent groups.

## Anode and Cathode

As the anode material, generally, a material having a large work function is preferably used so that holes can be smoothly injected into the organic material layer. Specific examples of the anode material include metals such as

vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or $SNO_2$:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or $LiO_2$/Al, and the like, but are not limited thereto.

In addition, a hole injection layer may be further included on the anode. The hole injection layer is composed of a hole injection material, and the hole injection material is preferably a compound which has an ability of transporting the holes, a hole injection effect in the anode and an excellent hole injection effect to the light emitting layer or the light emitting material, prevents movement of an exciton generated in the light emitting layer to the electron injection layer or the electron injection material, and has an excellent thin film forming ability.

It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

## Hole Transport Layer

The hole transport layer used in the present disclosure is a layer receiving holes from the hole injection layer which is formed on the anode or the cathode, and transporting the holes to the light emitting layer. The hole transport material is suitably a material having large mobility to the holes, which may receive holes from the anode or the hole injection layer and transfer the holes to the light emitting layer.

Preferably, the hole transport layer is formed by a solution process. Meanwhile, in the case of preparing the hole transport layer by a solution process, not only the layer should have a solubility in a solvent, but also after forming the hole transport layer, it should not be dissolved in a solvent used for forming other layers. For these reasons, the polymer-based materials are mainly used.

By the way, when the hole transport layer is formed of such a polymer material, the hole mobility is lower than that of a monomolecular material used in an existing deposition process. Therefore, it is also necessary to adjust the electron mobility of other functional layer, for example, electron transport layer, included in the organic light emitting device. As will be described later, in the present disclosure, the characteristics of the light emitting device can be improved by controlling the electron mobility of the electron transport layer.

## Light Emitting Layer

The light emitting material contained in the light emitting layer is a material that can receive holes and electrons from a hole transport layer and an electron transport layer, respectively, and combine the holes and the electrons to emit light in a visible ray region, and is preferably a material having good quantum efficiency to fluorescence or phosphorescence.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence. Specific examples of the light emitting material include an 8-hydroxy-quinoline aluminum complex ($Alq_3$); a carbazole-based compound; a dimerized styryl compound; BAlq; a 10-hydroxybenzoquinoline-metal compound; a benzoxazole, benzthiazole and benzimidazole-based compound; a poly(p-phenylenevinylene)(PPV)-based polymer; a spiro compound; polyfluorene, lubrene, and the like, but are not limited thereto.

The light emitting layer may include a host material and a dopant material. The host material may be a fused aromatic ring derivative, a heterocycle-containing compound or the like. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

Examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound

is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

**Electron Transport Layer**

The organic light emitting device according to the present disclosure may include an electron transport layer which receives electrons from a cathode and an electron injection layer and transports the electrons to an electron control layer.

The electron transport material is suitably a material which may receive electrons well from a cathode and transfer the electrons to a light emitting layer, and has a large mobility for electrons. However, when the hole transport layer is prepared by a solution process as described above, the hole mobility of the hole transport layer is slightly low and, therefore, when a material having a high mobility for electrons is used without considering these factors, it causes a significant difference in the amount of holes and electrons reaching the light emitting layer, which is a factor for reducing the lifetime of the organic light emitting device.

Therefore, in the present invention, by using a material having an electron mobility of $10^{-5}$ $cm^2/Vs$ or less as a material constituting the electron transport layer, the amount of holes and electrons reaching the light emitting layer is controlled to improve the lifetime of the organic light emitting device.

Preferably, the electron mobility of the material constituting the electron transport layer is $10^{-6}$ $cm^2/Vs$ or less, $10^{-7}$ $cm^2/Vs$ or less, $10^{-8}$ $cm^2/Vs$ or less, $10^{-9}$ $cm^2/Vs$ or less, $10^{-10}$ $cm^2/Vs$ or less, or $10^{-11}$ $cm^2/Vs$ or less. More preferably, the electron mobility of the material constituting the electron transport layer is $10^{-13}$ $cm^2/Vs$. Meanwhile, the method of measuring the electron mobility is widely known in the art, and as an example, it can be measured in the same manner as described in Experimental Examples below.

As an example of the material constituting the electron transport layer that satisfies the above conditions, a compound represented by the following Chemical Formula 1 may be used.

[Chemical Formula 1]

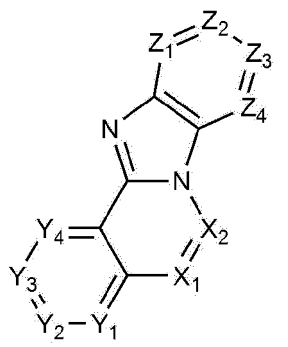

in Chemical Formula 1,

$X_1$ is $CR_1$ or N, $X_2$ is $CR_2$ or N,

$Y_1$ is $CR_5$ or N, $Y_2$ is $CR_6$ or N, $Y_3$ is $CR_7$ or N, $Y_4$ is $CR_8$ or N,

$Z_1$ is $CR_9$ or N, $Z_2$ is $CR_{10}$ or N, $Z_3$ is $CR_{11}$ or N, $Z_4$ is $CR_{12}$ or N,

$X_1$, $X_2$, $Y_1$ to $Y_4$ and $Z_1$ to $Z_4$ are not N at the same time,

$R_1$, $R_2$ and $R_5$ to $R_{12}$ are the same as or different from each other, and each independently, hydrogen; deuterium; a substituted or unsubstituted $C_{6-60}$ aryl; a substituted or unsubstituted $C_{2-60}$ heteroaryl containing any one or more heteroatoms selected from the group consisting of N, O, and S; or a substituted or unsubstituted phosphine oxide group, or two adjacent substituents of $R_1$, $R_2$ and $R_5$ to $R_{12}$ are bonded to each other to form a substituted or unsubstituted hydrocarbon ring; or a substituted or unsubstituted heterocycle.

Preferably, the Chemical Formula 1 is represented by any one of the following Chemical Formulas 1-1 to 1-4:

[Chemical Formula 1-1]

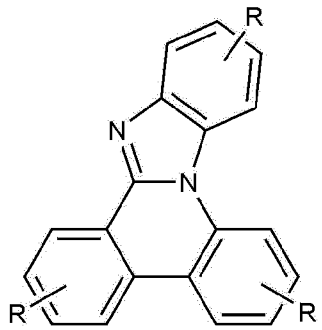

[Chemical Formula 1-2]

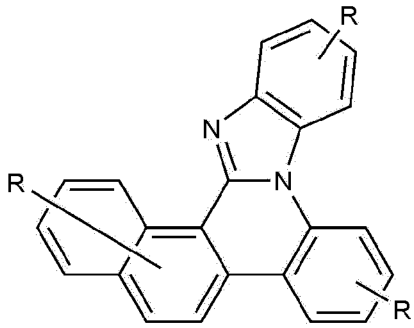

[Chemical Formula 1-3]

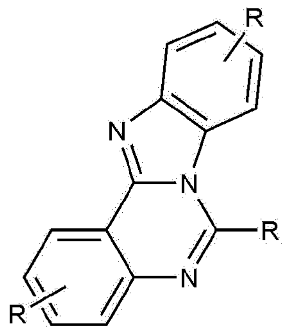

[Chemical Formula 1-4]

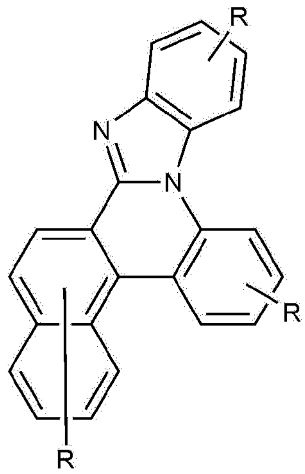

in Chemical Formulas 1-1 to 1-4,
one or two of R is -L-Ar, and the rest are hydrogen,
each L is independently a single bond; or a substituted or unsubstituted $C_{6-60}$ arylene,
Ar is a substituted or unsubstituted $C_{6-60}$ aryl; a substituted or unsubstituted $C_{2-60}$ heteroarylene containing any one or more selected from the group consisting of N, O and S; or -P(=O)($Ar_1$)($Ar_2$),
$Ar_1$ and $Ar_2$ are each independently a substituted or unsubstituted $C_{6-60}$ aryl; or a substituted or unsubstituted $C_{2-60}$ heteroarylene containing any one or more selected from the group consisting of N, O and S.

Preferably, L is a single bond; phenylene; naphthylene; or diphenylfluorenyl.

Preferably, Ar is phenyl, biphenylyl, terphenylyl, naphthyl, phenanthrenyl, anthracenyl, triphenylenyl, pyrenyl, pyridinyl, quinolinyl, or isoquinolinyl.

Preferably, $Ar_1$ and $Ar_2$ are each independently phenyl or naphthyl.

Representative examples of the compound represented by Chemical Formula 1 are as follows:

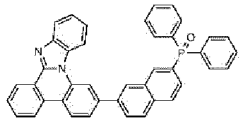
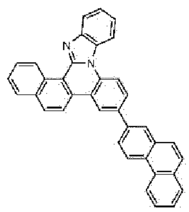
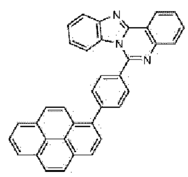
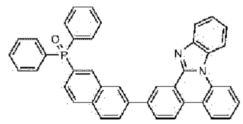
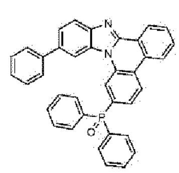
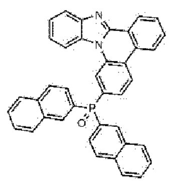
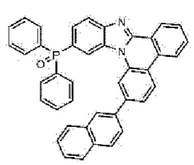
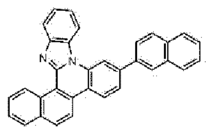
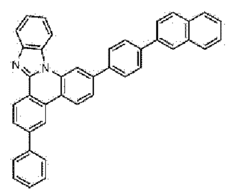
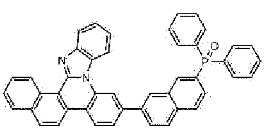
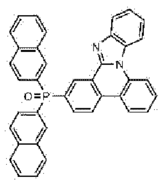
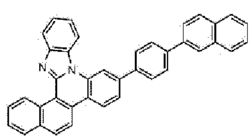

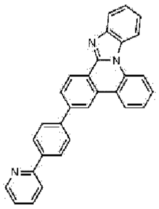
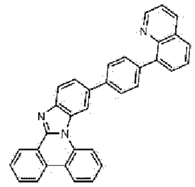
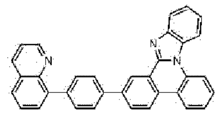
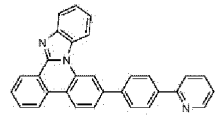
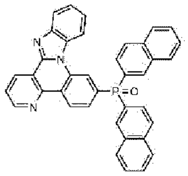
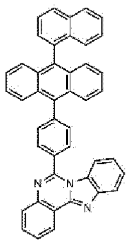
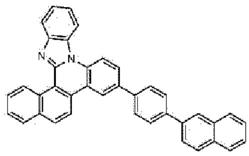
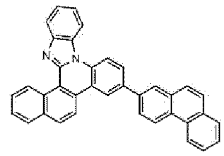
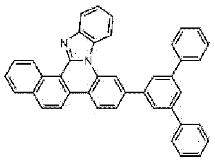
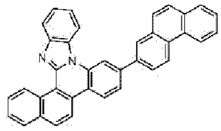
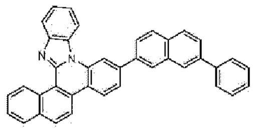
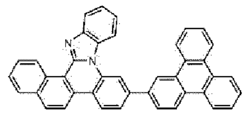
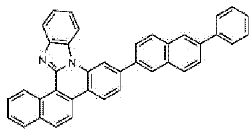
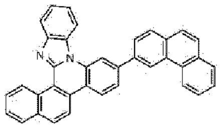
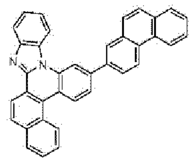
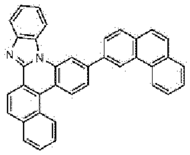
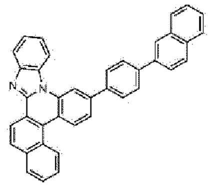
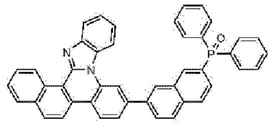
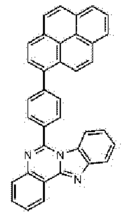
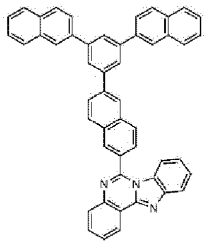
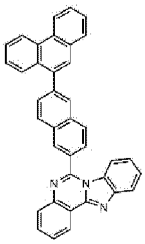
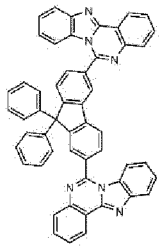
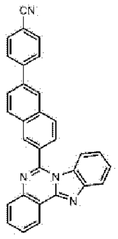
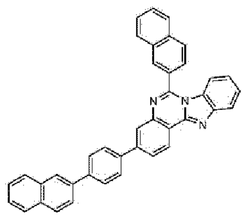

## Electron Injection Layer

The organic light emitting device according to the present disclosure may include an electron injection layer that injects electrons from the electrode.

As the electron injection material, a compound which has a capability of transporting the electrons, an electron injection effect from the cathode, and an excellent electron injection effect to the light emitting layer or the light emitting material, prevents movement of an exciton generated in the light emitting layer to the hole injection layer, and has an excellent thin film forming ability is preferable.

Specific examples thereof include fluorenone, anthraquinodimethane, diphenoquinone, thiopyran dioxide, oxazole, oxadiazole, triazole, imidazole, perylene tetracarboxylic acid, fluorenylidene methane, anthrone, and the like, and derivatives thereof, a metal complex compound, a nitrogen-containing 5-membered cycle derivative, and the like, but are not limited thereto. Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

**Organic Light Emitting Device**

The structure of the organic light emitting device according to an embodiment of the present disclosure is illustrated in FIGS. 1 and 2.

FIG. 1 shows an example of an organic light emitting device comprising a substrate 1, an anode 2, a light emitting layer 3, an electron transport layer 4 and a cathode 5. FIG. 2 shows an example of an organic light emitting device comprising a substrate 1, an anode 2, a hole injection layer 6, a hole transport layer 7, a light emitting layer 3, an electron transport layer 4, an electron injection layer 8, and a cathode 5.

The organic light emitting device according to the present disclosure can be manufactured by sequentially stacking the above-mentioned constitutional elements. In this case, the organic light emitting device may be manufactured by depositing a metal, metal oxides having conductivity, or an alloy thereof on a substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device may be manufactured by sequentially depositing a cathode material, an organic material layer and an anode material on a substrate.

In particular, in the present disclosure, the hole injection layer, the hole transport layer, and the light emitting layer may be formed by a solution process. Here, the solution process refers to a method of forming a layer by dissolving a material forming a functional layer in a solvent and then forming the functional layer and removing the solvent, and examples thereof include spin coating, dip coating, doctor blading, inkjet printing, screen printing, spray method, roll coating, and the like, but are not limited thereto.

Further, the electron transport layer is preferably formed by a sublimination process. The sublimination process refers to forming an electron transport layer by applying heat to a material forming the electron transport layer and evaporating it, and is also referred to as a thermal evaporation process.

In addition to such a method, the organic light emitting device may be manufactured by sequentially depositing a cathode material, an organic material layer, and an anode material on a substrate (WO 2003/012890). However, the manufacturing method is not limited thereto.

The organic light emitting device according to the present disclosure may be a front side emission type, a back side emission type, or a double side emission type according to the used material.

Hereinafter, preferred examples of the present disclosure will be provided for a better understanding of the invention. However, these examples are presented for illustrative purposes only, and the scope of the present invention is not limited thereto.

**EXAMPLE**

(1) Used Material

**HIL:** Ppy/Poly(TFE-PSEPVE)

64.6 g (7.38 mmol of Nafion™ monomer unit) of Nafion™ polymer dispersion, 125 g of deionized water, 62 mg of ferric sulfate (Aldrich, Cat.# 307718) and 0.175 mL (2.10 mmol) of 37% (w/w) aqueous hydrochloric acid solution (Ashland Chemicals; cat.# 3471440) were placed in a 500 mL reaction vessel equipped with an electrically controlled propeller-type stirring paddle, and the reaction mixture was stirred at 200 rpm. After stirring for 5 minutes, when 0.253 mL (3.58 mmol) of freshly distilled pyrrole (Acros Organics, cat.# 157711000) was added thereto, the color of the reaction mixture rapidly changed from transparent to dark green. After further stirring for 5 minutes, gradually injecting an oxidizing solution made of 1.01 g (4.24 mmol) of sodium persulfate (Fluka; cat.# 71889) in 10 mL of deionized water was initiated at a rate of 1.0 mL/h. This was performed by making a small tube from a 10 mL syringe on automatic syringe pump in the reaction vessel, with the end of the tube being about 4" above the reaction mixture. When an oxidizing agent was added to the reaction mixture, its color changed from dark green to greenish brown. It took about 10 hours to complete

the addition of the oxidizing agent solution. Both polymerization and addition of the oxidizing agent solution were carried out at room temperature. The particle size factor measured by Eccusizer (Model 780A; Particle Sizing Systems) until the end of the addition was 1.2 million particles in each 1 mL of the dispersion when the particles were larger than 0.75 μm.

The reaction mixture was further reacted for 7.5 hours, and then 15 g of Lewatit Monoplus S100, 15 g of Lewatit MP62WS, and 20 g of n-propanol were added. Lewatit Monoplus S100 is Bayer's trade name for sodium sulfonate of a crosslinked polystyrene ion exchange resin. Lewatit MP62WS is Bayer's trade name for tertiary/quaternary amine of a crosslinked polystyrene ion exchange resin. The resin was first washed with deionized water until it had no color in water before use.

The reaction mixture containing the resin was stirred for 4.5 hours, and then filtered through two Whatman #54 filter papers. The particle size factor was 750,000 particles in each 1 mL of the dispersion when the particles were larger than 0.75 μm. The dispersion was very stable without any sign of sedimentation. The pH of the dispersion was 5.4 when measured with a pH meter, and the conductivity of the dried film was $5.4 \times 10^{-6}$ S/cm. For the proportion of solids, a small amount of the dispersion was dried with a stream of nitrogen to form a solid film. This was measured to be 4.1%.

**HTL**

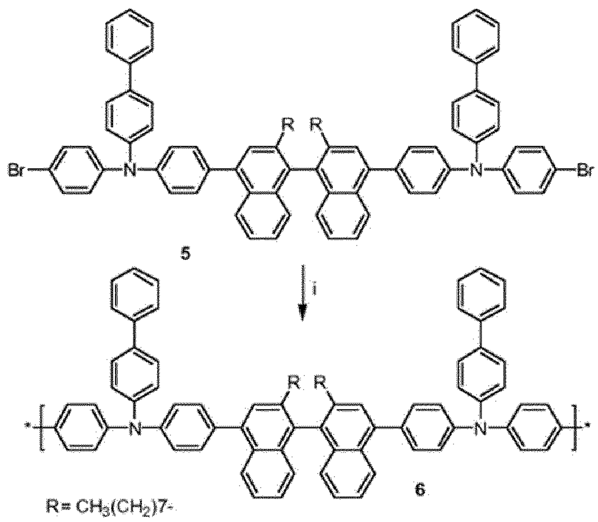


5

6

R= $CH_3(CH_2)_7$-

Monomer 5 (1.35 g, 1.06 mmol) was added to a scintillation vial and dissolved in toluene (13 mL). A clean, dried 50 mL Schlenk tube was charged with bis(1,5-cyclooctadiene)nickel(0) (0.597 g, 2.17 mmol). 2,2'-Dipyridyl (0.339 g, 2.17 mmol) and 1,5-cyclooctadiene (0.235 g, 2.02 mmol) were weighed and placed in a scintillation vial, and dissolved in N,N'-dimethylformamide (2 mL). The solution was added to a Schlenk tube. The Schlenk tube was inserted into an aluminum block, and the block was heated and stirred at a point where the internal temperature was set to be 60°C. The catalyst system was maintained at 60°C for 30 minutes and then raised to 70°C. A monomer solution in toluene was added to a Schlenk tube and the tube was sealed. The polymerization mixture was stirred at 70°C for 18 hours. Then, the Schlenk tube was removed from the block and cooled to room temperature. The tube was removed from the glove box and the contents were poured into a solution of conc. HCl/MeOH (1.5% v/v conc. HCl). After stirring for 2 hours, the polymer was collected by vacuum filtration and dried under vacuum. The polymer was purified by successive precipitations from toluene into HCl/MeOH (1% v/v conc. HCl), MeOH, toluene (CMOS grade), and 3-pentanone. A white fibrous polymer (1.1 g) was obtained. The molecular weight of the polymer was determined by GPC (THF mobile phase, polystyrene standard):

Mw=427,866; Mn=103,577

2) Manufacture of organic light emitting device

A patterned indium tin oxide (ITO) coated glass substrate from Thin Film Devices, Inc. was used. These ITOs were based on Corning 1737 glass coated with ITO with a sheet resistance of 30 ohm/square and a light transmittance of 80%. The patterned ITO substrate was ultrasonically cleaned in an aqueous detergent solution and rinsed with distilled water. Then, the patterned ITO was ultrasonically cleaned in acetone, rinsed with isopropanol, and dried under a nitrogen atmosphere. Then, it was treated with UV ozone for 10 minutes.

The aqueous dispersion of HIL was spin-coated on the prepared ITO (thickness: 1000 Å) and heated to remove the solvent, thereby preparing a hole injection layer having a thickness of 500 Å. After cooling, the toluene solution of HTL was spin-coated on the hole injection layer and heated to remove the solvent, thereby preparing a hole transport

layer having a thickness of 200 Å. After cooling, a methyl benzoate solution of the following Host compound and the following Emitter compound (weight ratio of 20:1) was spin-coated on the hole transport layer and heated to remove the solvent, thereby forming a light emitting layer having a thickness of 400 Å. The substrate prepared up to the light-emitting layer was placed in a vacuum chamber, and the following ETL1 compound was thermally deposited to form an electron transport layer having a thickness of 200 Å. LiF was deposited on the electron transport layer to form an electron injection layer having a thickness of 10 Å, and then aluminum having a thickness of 1000 Å was deposited, thereby manufacturing an organic light emitting device.

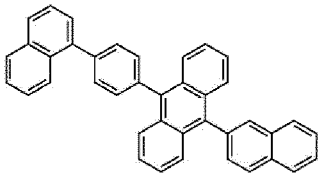

Host

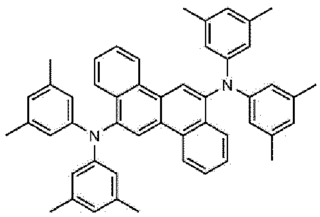

Emitter

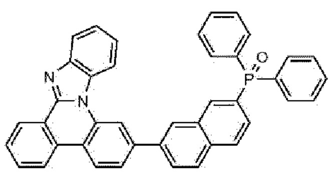

ETL1

**Examples 2 to 7 and Comparative Example**

The organic light-emitting devices were manufactured in the same manner as in Example 1, except that the compounds shown in Table 1 were used instead of the ETL1 compound. Each compound used in Table 1 is as follows.

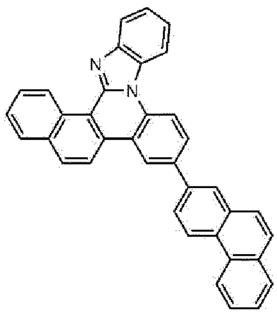

ETL2

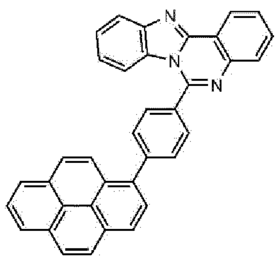

ETL3

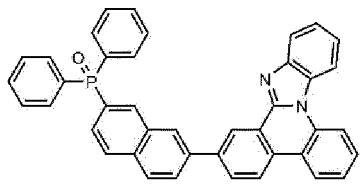

ETL4

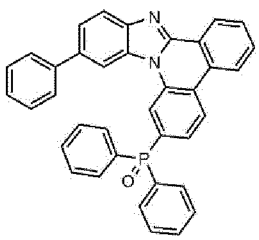

ETL5

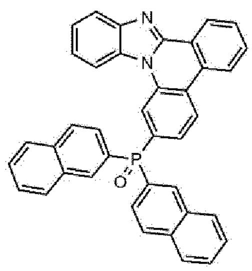

ETL6

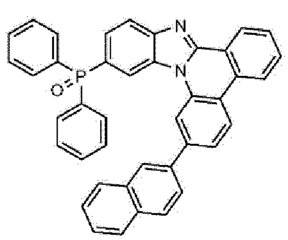

ETL7

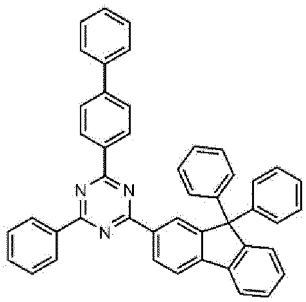

ETL-A

**Experimental Example**

(1) Measurement of Electron Mobility

Electron mobility was measured for the materials used to form the electron transport layer in the Examples and Comparative Example. Specifically, the following EOD (Electron Only Device) was manufactured.

EOD structure: ITO/N-CGL(200Å)/ETL(2000Å)/N-CGL(200Å)/Al(1000Å)

- N-CGL: formed in a thickness of 200Å by doping the following ET1 material with 2% Li
- ETL: The material whose electron mobility is to be measured was formed in a thickness of 2000Å

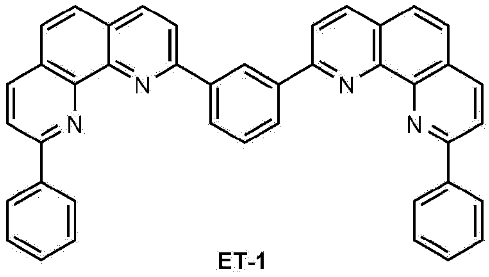

ET-1

The current according to voltage was measured for the EOD, and the electron mobility in an electric field of $2\times10^5$V/cm was calculated according to a Poole-Frenkel Equation below (APPLIED PHYSICS LETTERS 90, 203512 (2007)). The results are shown in Table 1 below.

**Poole-Frenkel Equation**

$$J = \frac{9}{8}\varepsilon\varepsilon_0\frac{F^2}{d}u_0\exp(\beta\sqrt{E}).$$

$$\mu(F) = \mu_0\exp(\beta\sqrt{F})$$

*Where,*

*$\varepsilon$: Relative dielectric constant ≈ 3*
*$\varepsilon_0$ : Permitivity in vaccum $8.85x10^{-14}$C/Vcm*
*d:Thickness of the ETL Layer(2000 Å)*
*$\beta$ : Pool-Frenkel constant (field lowing factor)*

(2) Performance Measurement of Organic Light Emitting Device

In addition, the driving voltage and efficiency of the organic light emitting devices prepared in the Examples and Comparative Example were measured at a current density of 10 mA/cm$^2$, and the lifetime was measured at a current density of 20 mA/cm$^2$, and the results are shown in Table 1 below. In Table 1 below, T80 means the time required for the luminance to be reduced to 80% of the initial luminance.

[Table 1]

| | Electron transport layer | Electron mobility ($cm^2/Vs$) | Driving voltage ($V@10mA/cm^2$) | Efficiency ($V@10mA/cm^2$) | T80 ($hr@20mA/cm^2$) |
|---|---|---|---|---|---|
| Example 1 | ETL1 | $7.43\times10^{-12}$ | 5.01 | 8.3 | 12450 |
| Example 2 | ETL2 | $5.11 \times 10^{-7}$ | 4.33 | 6.0 | 8920 |
| Example 3 | ETL3 | $2.63\times10^{-9}$ | 4.45 | 6.9 | 10300 |
| Example 4 | ETL4 | $6.27\times10^{-12}$ | 4.61 | 7.9 | 11800 |
| Example 5 | ETL5 | $1.31 \times 10^{-9}$ | 4.50 | 7.2 | 11320 |
| Example 6 | ETL6 | $2.30 \times 10^{-8}$ | 4.41 | 6.3 | 9300 |
| Example 7 | ETL7 | $2.94\times10^{-12}$ | 4.58 | 7.8 | 11000 |
| Comparative Example | ETL-A | $4.91 \times10^{-5}$ | 4.32 | 6.0 | 6740 |

**[Description of Symbols]**

| | | | |
|---|---|---|---|
| 1: | substrate | 2: | anode |
| 3: | hole injection layer | 4. | electron transport layer |
| 5: | cathode | 6: | hole injection layer |
| 7: | hole transport layer | 8: | electron injection layer |

## Claims

1. An organic light emitting device comprising:

   an anode;
   a cathode that is provided opposite to the anode;
   a light emitting layer that is provided between the anode and the cathode; and
   an electron transport layer that is provided between the light emitting layer and the cathode,
   wherein an electron mobility of a material constituting the electron transport layer is $10^{-5}$ $cm^2/Vs$ or less.

2. The organic light emitting device of claim 1, wherein an electron mobility of a material constituting the electron transport layer is $10^{-7}$ $cm^2/Vs$ or less.

3. The organic light emitting device of claim 1, wherein an electron mobility of a material constituting the electron transport layer is $10^{-8}$ $cm^2/Vs$ or less.

4. The organic light emitting device of claim 1, wherein the light emitting layer is formed by a solution process.

5. The organic light emitting device of claim 1, wherein the electron transport layer is adjacent to the light emitting layer.

6. The organic light emitting device of claim 1, wherein the electron transport layer is formed by a sublimation process.

7. The organic light emitting device of claim 1, wherein a hole transport layer is included between the anode and the light emitting layer, and the hole transport layer is formed by a solution process.

8. The organic light emitting device of claim 1, wherein the material constituting the electron transport layer is a compound represented by the following Chemical

Formula 1:

[Chemical Formula 1]

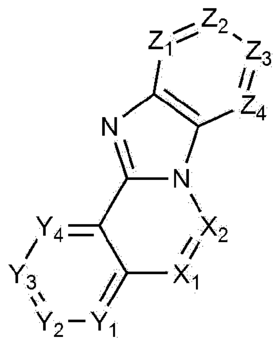

in Chemical Formula 1,

$X_1$ is $CR_1$ or N, $X_2$ is $CR_2$ or N,
$Y_1$ is $CR_5$ or N, $Y_2$ is $CR_6$ or N, $Y_3$ is $CR_7$ or N, $Y_4$ is $CR_8$ or N,
$Z_1$ is $CR_9$ or N, $Z_2$ is $CR_{10}$ or N, $Z_3$ is $CR_{11}$ or N, $Z_4$ is $CR_{12}$ or N,
$X_1$, $X_2$, $Y_1$ to $Y_4$ and $Z_1$ to $Z_4$ are not N at the same time,
$R_1$, $R_2$ and $R_5$ to $R_{12}$ are the same as or different from each other, and each independently, hydrogen; deuterium; a substituted or unsubstituted $C_{6-60}$ aryl; a substituted or unsubstituted $C_{2-60}$ heteroaryl containing any one or more heteroatoms selected from the group consisting of N, O, and S; or a substituted or unsubstituted phosphine oxide group, or two adjacent substituents of $R_1$, $R_2$ and $R_5$ to $R_{12}$ are bonded to each other to form a substituted or unsubstituted hydrocarbon ring; or a substituted or unsubstituted heterocycle.

**9.** The organic light emitting device of claim 8,
wherein the compound represented by Chemical Formula 1 is any one selected from the group consisting of:

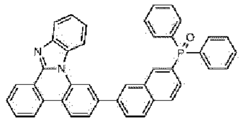
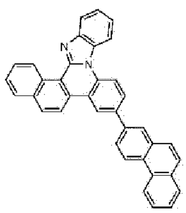
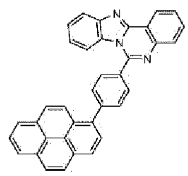
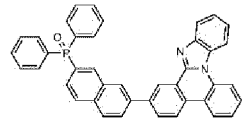
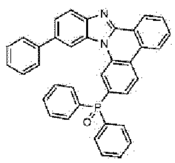
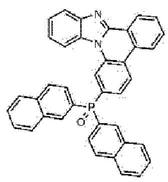
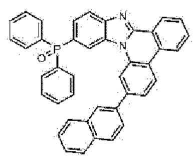
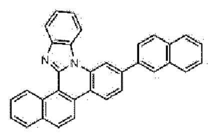
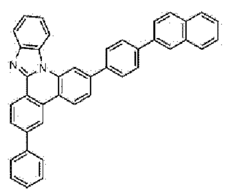
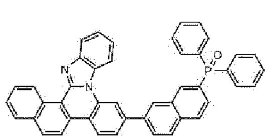
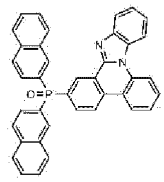
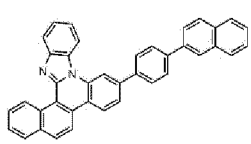

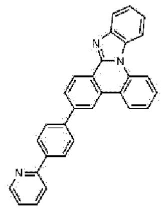
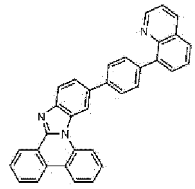
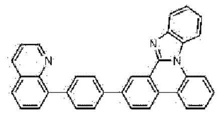
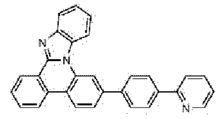
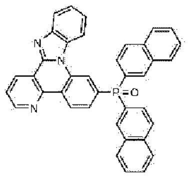
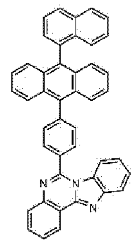
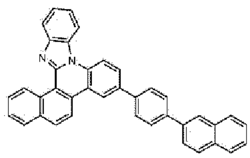
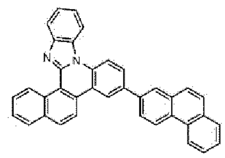
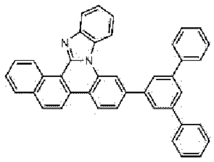
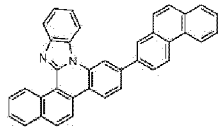
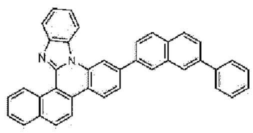
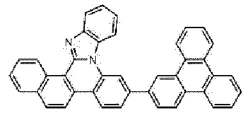
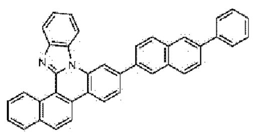
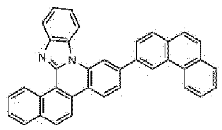
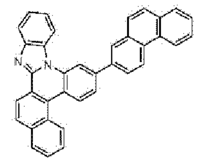
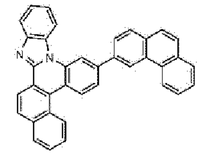
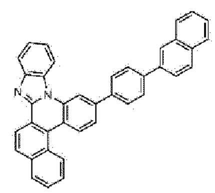
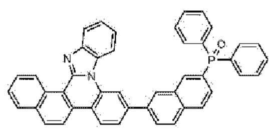
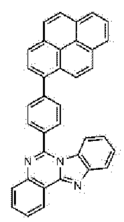
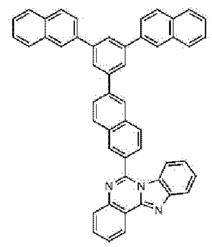
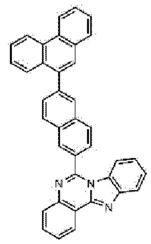
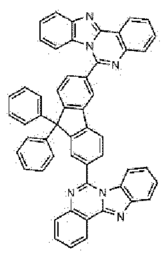
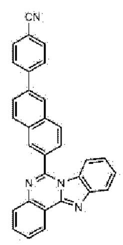
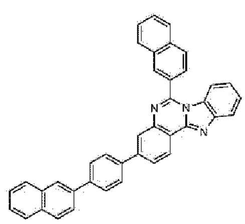

**10.** A method for manufacturing an organic light emitting device comprising the steps of:

1) forming a light emitting layer on an anode by a solution process;
2) forming an electron transport layer on the light emitting layer by a sublimation process; and
3) depositing a cathode on the electron transport layer,

wherein an electron mobility of a material constituting the electron transport layer is $10^{-5}$ $cm^2/Vs$ or less,

**11.** The method for manufacturing an organic light emitting device according to claim 10, prior to step 1), further comprising the step of forming a hole transport layer on the anode by a solution process.

[FIG. 1]

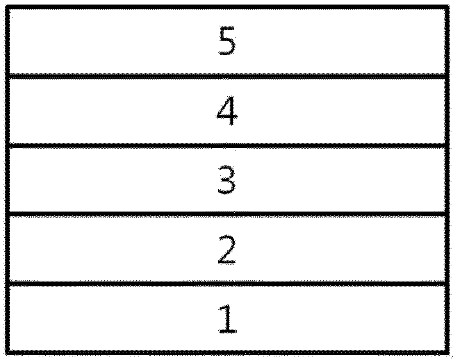


[FIG. 2]

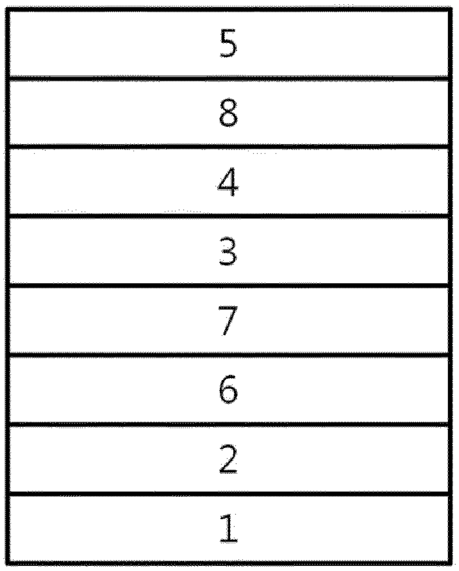

# INTERNATIONAL SEARCH REPORT

International application No.
**PCT/KR2019/010418**

A. CLASSIFICATION OF SUBJECT MATTER

***H01L 51/50(2006.01)i, H01L 51/56(2006.01)i, H01L 51/00(2006.01)i***

According to International Patent Classification (IPC) or to both national classification and IPC

B. FIELDS SEARCHED

Minimum documentation searched (classification system followed by classification symbols)
H01L 51/50; C01D 15/00; C01G 39/00; C07D 235/08; C07D 491/048; C07D 495/04; C09B 57/00; C09K 11/06; H01L 51/56; H01L 51/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: organic light emitting device, electron transport layer, electron mobility, solution process, evaporation process

C. DOCUMENTS CONSIDERED TO BE RELEVANT

| Category* | Citation of document, with indication, where appropriate, of the relevant passages | Relevant to claim No. |
|---|---|---|
| X<br><br>Y | KR 10-2009-0131550 A (SAMSUNG MOBILE DISPLAY CO., LTD.) 29 December 2009<br>See paragraphs [0032]-[0043], [0053], [0055], [0075], [0077]; and figure 2. | 1-7,10-11<br><br>8-9 |
| Y | KR 10-2016-0005196 A (SAMSUNG DISPLAY CO., LTD.) 14 January 2016<br>See claims 15, 17. | 8-9 |
| A | WO 2017-221999 A1 (IDEMITSU KOSAN CO., LTD.) 28 December 2017<br>See paragraphs [0021], [0100], [0138], [0142]. | 1-11 |
| A | JP 2017-147373 A (IDEMITSU KOSAN CO., LTD.) 24 August 2017<br>See paragraphs [0058]-[0061], [0076]. | 1-11 |
| A | KR 10-2017-0048611 A (MERCK PATENT GMBH.) 08 May 2017<br>See claims 1, 4. | 1-11 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 DECEMBER 2019 (04.12.2019) | **04 DECEMBER 2019 (04.12.2019)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.
**PCT/KR2019/010418**

| Patent document cited in search report | Publication date | Patent family member | Publication date |
|---|---|---|---|
| KR 10-2009-0131550 A | 29/12/2009 | US 2009-0315024 A1 | 24/12/2009 |
| KR 10-2016-0005196 A | 14/01/2016 | US 10193078 B2 | 29/01/2019 |
| | | US 2016-0005980 A1 | 07/01/2016 |
| WO 2017-221999 A1 | 28/12/2017 | CN 109311904 A | 05/02/2019 |
| | | JP 2019-521995 A | 08/08/2019 |
| | | KR 10-2019-0020683 A | 04/03/2019 |
| | | US 2019-0161497 A1 | 30/05/2019 |
| JP 2017-147373 A | 24/08/2017 | US 2017-0244044 A1 | 24/08/2017 |
| KR 10-2017-0048611 A | 08/05/2017 | CN 102498587 A | 13/06/2012 |
| | | CN 106058064 A | 26/10/2016 |
| | | CN 106058064 B | 07/05/2019 |
| | | DE 102009041289 A1 | 17/03/2011 |
| | | JP 2013-504882 A | 07/02/2013 |
| | | JP 2015-164204 A | 10/09/2015 |
| | | JP 6490480 B2 | 27/03/2019 |
| | | KR 10-2012-0080606 A | 17/07/2012 |
| | | TW 201129245 A | 16/08/2011 |
| | | US 2012-0168735 A1 | 05/07/2012 |
| | | WO 2011-032624 A1 | 24/03/2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020180099501
- KR 1020000051826
- WO 2003012890 A


### Non-patent literature cited in the description


- *APPLIED PHYSICS LETTERS,* 2007, vol. 90, 203512